# EUROPEAN PATENT SPECIFICATION

(11) **EP 1 904 038 B1**
(45) Date of publication and mention of the grant of the patent: **16.11.2011**
(21) Application number: 06770884.2
(22) Date of filing: 19.05.2006
(51) Int. Cl.: A61K 9/26

(54) **COMPRESSED PHARMACEUTICAL COMPOSITION COMPRISING COATED PELLETS AND A DIRECT COMPRESSION MIXTURE, AND METHOD OF PREPARATION THEREOF**
KOMPRIMIERTE PHARMAZEUTISCHE ZUSAMMENSETZUNG MIT BESCHICHTETEN PELLETS UND DIREKTES KOMPRESSIONSGEMISCH UND HERSTELLUNGSVERFAHREN DAFÜR
COMPOSITION PHARMACEUTIQUES COMPRIMEE COMPRENANT DES GRANULES ENROBES ET UN MELANGE A COMPRESSION DIRECTE, ET PROCEDE DE PREPARATION

(30) Priority: 20.05.2005 US 133864
(43) Date of publication of application: 02.04.2008
(73) Proprietor: Actavis Group HF, 220 Hafnarfirdi (IS)
(72) Inventor: FENG, Hengsheng, Cherry Hill, NJ 08003 (US)
(74) Representative: Matthews, Derek Peter
(86) International application number: PCT/US2006/019801
(87) International publication number: WO 2006/127637

(56) References cited:
- WO-A-89/02742
- WO-A-99/32092
- US-A- 4 882 167
- US-A1- 2004 081 695

## Description

### BACKGROUND OF THE INVENTION

Modified- or controlled-release pharmaceutical dosage forms provide the advantages of releasing a pharmaceutical agent to a patient in a controlled manner. Controlled-release may include sustained-, delayed- or pulsed-release at a particular time. Alternatively, controlled may mean extended-release of the pharmaceutical agent allowing for the reduction in the number of doses the patient is administered in a given timeframe. Such controlled-release can prevent "dose dumping", extreme fluctuations in the plasma concentration of the pharmaceutical agent, and the like.

Compared to a conventional tablet, pellets in tablets provide the following benefits: the ability to carry a large dose of pharmaceutical agent, the ability to obtain a wide range of release profiles, a reduction in dose to dose variability, more reliable dose to dose release, and a reduction in bio-variability. Pellets allow for ease and convenience of manufacturing as they can be further processed into a capsule or a tablet.

To prepare solid dosage forms, compression is the most favorable process. Despite its advantages, such as reduced processing time and cost, the success of compression is determined by the chemical and physical properties of the mass carrying the pharmaceutical agent, choice of fillers, behavior of the mixture during process and the desired properties of the final dosage form (e.g. release properties). The difficulty in formulation is increased when the number of pharmaceutical agents is increased, the form of the pharmaceutical agent (e.g. powder versus granules, pellets, beads, etc.), whether the pharmaceutical agent is coated or formulated to have a particular size, shape, or other property.

The following literature discusses the challenges of preparing compression dosage forms from coated granules or discrete units of pharmaceutical agents:

U.S. Patent No. 4,874,614 generally discloses a method of preventing the fracture of coated drug granules during a compression of the granules into a tablet matrix, wherein the drug release of the tablet decreases with time while increasing the amount of microcrystalline cellulose in the dosage form.

U.S. Patent No. 5,780,055 generally discloses "cushioning beads" prepared from microcrystalline cellulose and a disintegrant such as croscarmellose sodium. The cushioning beads are prepared to be mechanically weaker than the coated beads containing active ingredients. The cushioning beads cushion the coated beads from damage when compacted. The disclosed beads had an average diameter of about 0.2-2.0 mm and preferably about 0.5-1.5 mm.
WO 99/32092 is directed to bite-dispersion tablets which disperse easily and quickly in the oral cavity. The drug is blended with excipients and a waxy material and compressed into tablets. The reference discloses examples of tablets having amounts of waxy material which are similar to the amounts of disintegrant.
U.S. Patent Application No. 2004/081695 is directed to an immediate release tablet formed by direct compression, and having an active ingredient and a powdered wax. The powdered wax is mixed or granulated directly with the active agent, nor does the reference describe the active agent as being in the form of coated pellets.
U.S. Patent No. 4,882,167 is directed to directly compressed products comprising an admixture of a fatty acid material or a neutral lipid, a wax and a hydrophobic carbohydrate polymer. The reference teaches that coated or encapsulated active agents have disadvantages, consequently the tablets do not have an encapsulated structure.
WO 89/02742 discloses a compressed tablet comprising an active agent, carnauba wax, a disintegrant, and additional excipients. It likewise does not disclose a compressed pharmaceutical composition comprising a plurality of coated pellets.

GB 1 598 458 generally discloses compression tableting of microcapsules with 2-20 % w/w of a water-soluble wax.

EP 1 131 057 generally discloses cushioning beads made from a microcrystalline hydrocarbon wax or a natural wax in an amount of at least 30 % by weight of the cushioning beads. The beads are used to prepare solid shaped articles containing biologically active ingredients by compression. To minimize the occurrence of segregation between active ingredient-loaded pellets and the cushioning beads, the '057 patent discloses that the inert beads should be of the same size, and approximately the same density as the active pellets. The cushioning beads are described by an average particle size of about 0.5 to about 2.0 mm and most preferably from 0.75 to 1.25 mm. Furthermore, the '057 patent distinguishes beads or pellets from granules, as pelletization is an agglomeration process that converts fine powders or granules into small, free-flowing, spherical or semi-spherical units. It is additionally stated that, as opposed to the process of granulation, the production of beads results in a narrow size-range distribution.

### BRIEF SUMMARY OF THE INVENTION

Disclosed herein are compressed pharmaceutical compositions comprising a plurality of coated pellets, wherein the coated pellets comprise a pharmaceutically active agent or salt, solvate, hydrate, or polymorph thereof; and a compressible mixture, wherein the compressible mixture comprises i) a non-water soluble waxy filler and ii) a disintegrant, wherein the weight ratio of waxy filler to disintegrant is 15:1 to 50:1 and wherein the compressible mixture is in powder form with an average particle diameter of 0.1 to 175 micrometers, with the proviso that the compressible mixture is not prepared by extrusion, spheronization, high shear mixing, melt blending, melt pelletization, freeze-drying, or a combination thereof, and wherein the compressed composition exhibits an in vitro dissolution profile according to a USP compendia method that is substantially the same as the dissolution of the coated pellets in the absence of the compressible mixture.

In another embodiment, a process of making a compressed pharmaceutical composition comprises mixing a powdered, waxy filler and a disintegrant to form a compressible mixture with an average particle diameter of 0.1 to 175 micrometers, wherein the waxy filler is non-water soluble and wherein the weight ratio of waxy filler to disintegrant is 15:1 to 50:1, with the proviso that the compressible mixture is not prepared by extrusion, spheronization, high shear mixing, melt blending, melt pelletization, freeze-drying, or a combination thereof; mixing the compressible mixture with a plurality of coated pellets to form a pellet mixture, wherein the coated pellets comprise a pharmaceutically active agent or salt, solvate, hydrate, or polymorph thereof; and compressing the pellet mixture with conventional direct compression equipment, wherein the compression force is not more than about 25 kiloNewtons.

In another embodiment, a direct compression compressible mixture for forming compressed pharmaceutical compositions comprises a mixture of a powdered waxy filler and a disintegrant, wherein the weight ratio of waxy filler to disintegrant is 15:1 to 50:1, and optionally further comprising a cellulose filler, a binder, a lubricant, a glidant, a compression aid, a colorant, a preservative, a flavor, or combinations thereof, wherein the mixture has an average particle diameter of 0.1 to 175 micrometers, with the proviso that the compressible mixture is not prepared by extrusion, spheronization, high shear mixing, melt blending, melt pelletization, freeze-drying, or a combination; and wherein the compressible mixture is suitable for directly compressing mixtures of the compressible mixture and coated drug pellets with substantially no damage to the coated drug pellets.

### BRIEF DESCRIPTION OF THE DRAWINGS

Figure 1. Dissolution comparison for diltiazem HCl tablets and pellets

Figure 2. Dissolution comparison for diltiazem HCl tablets and pellets

Figure 3. Dissolution comparison for morphine sulfate tablets and pellets

Figure 4. Dissolution comparison for amphetamine tablets and pellets

Figure 5. Dissolution comparison for diltiazem HCl pellets and tablets prepared from wax and microcrystalline cellulose,

### DETAILED DESCRIPTION OF THE INVENTION

Disclosed herein are compressible mixtures which, when directly compressed with coated drug pellets, provide excellent protection of the pellet and pellet coating while at the same time providing a compressed pharmaceutical composition (e.g., tablet) having adequate hardness and low friability. As a result of the minimal damage to the pellet or pellet coating and the particular choice of mixture components, the resulting compressed pharmaceutical compositions exhibit a dissolution profile substantially the same as the dissolution profile of the coated drug pellets alone. Furthermore, the compressible mixtures blended with drug pellets can be used with conventional tableting equipment and require no special tools or machinery.

When the compressible mixture is prepared from a waxy filler, segregation is not observed between the drug pellets and the compressible mixture even when the waxy filler is in powdered form.

Also disclosed herein are compressed pharmaceutical compositions prepared from direct compression of a blend of compressible mixture and drug pellets. Methods of tableting the blend are also provided.

As used herein a "drug pellet" means subunits of a dosage form comprising an active agent in combination with a binder and other optional inert ingredients that have been formed into particles, pellets, granules, spheres or spheroids, beads, etc., (collectively referred to herein as "pellets").

As used herein a "coated pellet" or "coated drug pellet" means a pellet as defined above, which has been further coated with a functional or non-functional coating as described herein.

The compressible mixture used to prepare compressed pharmaceutical compositions containing drug pellets comprises a compression filler which is a waxy filler, a cellulose filler, or a mixture thereof and a disintegrant, specifically a "super" disintegrant. The compressible mixture is provided as a powdery or particulate material and is not further processed or compressed prior to combining with a pellet and subsequent compression into a compressed pharmaceutical composition.

As used herein, a "tablet", "compressed tablet", or "compressed pharmaceutical composition" mean the same unless otherwise indicated.

The compression filler (waxy filler, cellulose filler or a mixture thereof) provides a variety of functions. First, it protects the coated pellets during the compression process by absorbing the compressive forces thereby relieving or removing compressive force on the coated pellets. The resulting compressed pharmaceutical composition exhibits minimal damage to the pellets. Second, it acts as a binder to hold the pellets together once compressed. The resulting compressed pharmaceutical composition exhibits excellent hardness and minimal friability even in the absence of additional binders.

Exemplary waxy fillers include waxes such as carnauba wax (from the palm tree *Copernicia Cerifera*), vegetable wax, fruit wax, microcrystalline wax ("petroleum wax"), bees wax (white or bleached, and yellow), hydrocarbon wax, paraffin wax, cetyl esters wax, non-ionic emulsifying wax, anionic emulsifying wax, candelilla wax, combinations thereof, and the like. Other suitable waxy fillers include, for example, stearyl alcohol, cetyl alcohol, cetostearyl alcohol, polyethylene glycol (PEG) having a molecular weight of greater than about 3000 number average molecular weight, Mₙ (e.g. PEG 3350, PEG 4000, PEG 4600, PEG 6000, and PEG 8000). Each wax described herein can be in powder or flake form.

The waxy filler can specifically be a solid, hydrophobic material (i.e non-water soluble) or solid hydrophilic material (e.g. polyethylene glycols described above which are water soluble), but specifically a solid, hydrophobic material in powder form.

The waxy material can be in the form of a powder or flake. When in the form of a powder, the waxy material can have an average particle diameter of up to about 175 micrometers, specifically an average particle diameter of about 0.1 micrometers to about 150 micrometers, more specifically about 1.0 micrometers to about 100 micrometers, and yet more specifically about 10 to about 75 micrometers. When in the form of flakes, the waxy material can be milled into desired sizes and sieved using mesh filters. When milled, the waxy material can be maintained at a temperature and/or at low shear to prevent melting and agglomeration.

The melting point of the waxy material may be at any temperature above room temperature, specifically about 30 to about 150°C, more specifically about 75 to about 100 °C, and yet more specifically about 75 to about 90°C.

The cellulose filler can be any cellulose material that can provide a direct compressed pharmaceutical composition containing coated pellets where there is substantially no damage to the pellets or pellet coating. Exemplary cellulose fillers include powdered cellulose having a "cottony" or "fluffy" characteristic (non-flowing), or microcrystalline cellulose (Avicel PH microcrystalline celluoses, e.g. Avicel PH-102),

The amount of waxy filler, cellulose filler or a combination thereof that can be used in the compressed pharmaceutical composition (total weight of the drug pellets and compressible mixture) can be up to about 75 weight percent based on the total amount of the composition, specifically about 10 to 70 weight percent, more specifically about 20 to about 60 weight percent, and yet more specifically about 30 to about 50 weight percent based on the total amount of the compressed pharmaceutical composition.

The disintegrant present in the compressible mixture is used to facilitate the breakdown of the compressed or compacted compressible mixture in a fluid environment, specifically aqueous environments. The choice and amount of disintegrant can be tailored to ensure the dissolution profile of the tablet is substantially the same as the dissolution profile of the drug pellets alone. In an alternative embodiment, the choice and amount of disintegrant is tailored to provide additional release-retarding properties for those formulations where additional controlled-release is desired.

The disintegrants used in combination with the waxy filler or cellulose filler to form the compressible mixture include so-called "super" disintegrants known in the art. Exemplary super disintegrants include, for example, cross-linked sodium carboxymethylcellulose ("croscarmellose sodium", i.e., Ac-Di-Sol® available from FMC BioPolymer of Philadelphia, PA); crosslinked homopolymer of N-vinyl-2-pyrrolidone ("crospovidone", e.g., Polyplasdone® XL, Polyplasdone® XL-10, and Polyplasdone® INF-10 available from International Specialty Products, Wayne NJ); modified starches, such as sodium carboxymethyl starch, sodium starch glycolate (e.g., Primogel), and the like; alginates; and combinations thereof.

The amount of disintegrant used can be about 1 to about 10 weight percent based on the total weight of the compressed pharmaceutical composition, specifically about 2 to about 7 weight percent, and yet more specifically about 3 to about 5 weight percent. The ratio of compression filler to disintegrant is 15-1 to 50:1, specifically about 20:1 to about 45:1, and yet more specifically about 25:1 to about 40:1.

In addition to the super disintegrants, additional disintegrants that generally possess the ability to swell or expand upon exposure to the fluid environment, especially an aqueous environment can be used alone or in combination with the super disintegrants. Examples of such disintegrants are starch, and pregelatinized starch (e.g., Starch 1500® available from Colorcon).

In another embodiment, a combination of waxy and cellulose fillers can be used to allow for the tailoring of the resulting compressed pharmaceutical composition properties. For example, the compressible mixture containing only a waxy filler as the compression filler, provides a compressed pharmaceutical composition that is easily broken by hand (i.e. with finger pressure) without the presence of a score line on the compressed pharmaceutical composition. Such a compressed pharmaceutical composition still exhibits adequate hardness and can be packaged in bottles or in blister packs, and the like. Optionally, a coating can be provided if more protection to the compressed pharmaceutical composition is desired. To provide a harder compressed pharmaceutical composition while not damaging the coated drug pellets during compression varying amounts of a cellulose filler (e.g. microcrystalline cellulose) can be added to the compressible mixture in place of a portion of the waxy filler. The resulting compressed pharmaceutical composition is harder and less friable.

When the compression filler is a combination of a waxy filler and a cellulose filler, the ratio of the two components can be about (weight/weight) 100:0 to about 0:100; about 95:5 to about 5:95; about 90:10 to about 10:90; about 85:15 to about 15:85; about 80:20 to about 20:80; about 75:25 to about 25:75; about 70:30 to about 30:70; about 60:40 to about 40: 60; or about 45:65 to about 65:45.

Optionally, the compressible mixture can further comprise additional pharmaceutically acceptable excipients so long as the excipients do not result in significant damage the drug pellet coatings when compressed to form a compressed pharmaceutical composition. The optional additional excipients can provide desired properties, such as increased hardness of the resulting tablet, ease of manufacture of the tablet, enhancement of the taste and visual aspects of the tablet, enhanced stability, enhanced patient acceptability, etc. Pharmaceutical excipients include binders, lubricants, glidants, compression aids, colorants, preservatives, flavors, etc.

Additional optional excipients include, for example, silicified microcrystalline cellulose, powdered cellulose, polyvinyl pyrrolidone, hydroxypropyl cellulose, hydroxypropyl methylcellulose, methylcellulose hydroxyethyl cellulose, mannitol, sorbitol, lactose, digestible sugars, sucrose, liquid glucose, sorbitol, dextrose, isomalt, liquid maltitol, aspartame, lactose, talc, and the like, and combinations thereof.

The compressible mixture can further comprise a lubricant and/or glidant to aid in the tableting process. Exemplary lubricants include stearic acid, stearates (e.g., calcium stearate, magnesium stearate, and zinc stearate), sodium stearyl fumarate, glycerol behenate, mineral oil, polyethylene glycol, talc, vegetable oil, and combinations thereof. Glidants include, for example, silicon dioxide (e.g. fumed or colloidal). Certain materials can function both as a glidant and a lubricant.

The lubricant or glidant can be used in amounts of about 0.1 to about 15 weight percent of the total weight of the compressed pharmaceutical composition; specifically about 0.5 to about 5 weight percent; and yet more specifically about 0.75 to about 3 weight percent.

The colorants can include pharmaceutically acceptable dyes, pigments, and the like. Exemplary colorants include FD&C blues, greens, oranges, reds, yellows, lakes and the like; D&C blues, greens, oranges, reds, yellows, lakes, and the like; titanium dioxide; combinations thereof; and the like.

Typically the compressible mixture is prepared by merely mixing the waxy filler or cellulose filler in powder form with the disintegrant and optional additives. Care is taken to maintain the powder form of the waxy filler by keeping the mixture below the melting temperature of the waxy filler and by minimizing the shear on the mixture. The compressible mixture is thereby provided in powder form having average particle diameters of up to about 175 micrometers, specifically of about 0.1 micrometer to about 150 micrometers, more specifically about 1.0 micrometer to about 100 micrometers, and more specifically about 10 micrometers to about 75 micrometers. The compressible mixture is not formed by any of the following processes: extrusion, spheronization, high shear mixing, melt blending, melt pelletization, freeze-drying, or any combination thereof; nor is the compressible mixture prepared into beads.

It was unexpectedly discovered that the powdery compressible mixture containing the waxy filler in quantities as provided herein is able to provide direct compressed pharmaceutical compositions containing coated pellets where the pellets remain substantially undamaged. Furthermore, the resulting compressed pharmaceutical composition exhibits substantially no change in the dissolution characteristics as compared to the drug coated pellets alone. Such a result was highly unexpected as it is known that so-called "cushioning beads" prepared from wax can provide some protection to coated pellets during a direct compression tableting process. It has been previously disclosed that in order to avoid segregation between the cushioning beads and the drug pellets, that the beads and pellets should be about the same size. It was unexpectedly found that the compressible mixture prepared from a powdered waxy filler and disintegrant does not segregate when tableted in conventional tableting equipment. Not wishing to be bound by theory, but it is believed that the powdered waxy filler does not have good flowing properties which in effect retards segregation of the compressible mixture and the drug pellets.

The drug pellet can be prepared from any known pharmaceutically active agent, vitamin, dietary supplement, and the like, and is not limited thereby. The term "drug" or "active agent" is meant to include solvates (including hydrates) of the free compound or salt, crystalline and non-crystalline forms, as well as various polymorphs. Unless otherwise specified, the term "active agent" is used herein to indicate the active agent or a pharmaceutically acceptable salt thereof including any and all optical isomers, either alone or in combination.

Classes of pharmaceutically active agents that can be used in the pellets include, for example, alpha-2 adrenergic agents, analgesics, angiotensin-converting enzyme (ACE) inhibitors, antianxiety agents, antiarrhythmics, antibacterials, antibiotics, antidepressants, antidiabetics, antiemetics, antiepileptics, antifungal antihelminthics, antihistamines, antihyperlipidemics, antihypertensive agents, antiinfectives, antimalarials, antimicrobials, antimigraine agents, antimuscarinic agents, antineoplastic agents, antiprotozoal agents, antipsychotic agents, antispasmodics, antiviral agents, attention-deficit hyperactivity disorder (ADHD) agents, β-blockers, calcium channel blockers, chemotherapeutic agents, cholinesterase inhibitors, Cox-2 inhibitors, decongestants, diuretics, histamine-2 receptor antagonists, hypnotics, hypotensive agents, immunosuppresants, lipotropics, neuroleptics, opioid analgesics, peripheral vasodilators/vasoconstrictors, sedatives, serotonin receptor agonists, and the like.

Exemplary pharmaceutically active agents include amphetamine, dextroamphetamine, diltiazem, fluvastatin, hydromorphone, morphine, oxybutynin, oxycodone, paroxetine, propranolol, tolterodine, venlafaxine, their pharmaceutically acceptable salts, solvates, hydrates, and polymorphs.

In one embodiment, the coated drug pellets are the diltiazem pellets disclosed in U.S. Patent Nos. 4,894,240 to Geoghegan et al., 5,002,776 to Geoghegan et al., 5,286,497 to Hendrickson et al., 5,364,620 to Geoghegan et al., 5,439,689 to Hendrickson et al., 5,470,584 to Hendrickson et al., 6,214,385 to Heinicke et al., 6,033,687 to Heinicke et al. and 5,834,024 to Heinicke et al. the teachings of which are incorporated herein by reference.

In another embodiment, the coated drug pellets are the oxbutynin drug releasing beads disclosed in U.S. Patent No. 6,262,115 to Guittard et al., the teachings of which are incorporated herein by reference.

In yet another embodiment, the coated drug pellets are the sustained-release morphine particles disclosed in U.S. Patent No. 6,066,339 to Stark et al. and the pellets of morphine disclosed in U.S. Patent Nos. 5,202,128 and 5,378,474 to Morella et al., the teachings of which are incorporated herein by reference.

In one embodiment, the drug pellets and coated drug pellets are the fluvastatin pellets disclosed in U.S. Patent No. 5,356,896 to Kabadi et al. and the particles disclosed in U.S. Patent No. 6,242,003 to Kalb et al, the teachings of which are incorporated herein by reference.

In another embodiment, the drug pellets and coated drug pellets are the oxycodone pellets disclosed in U.S. Patent No. 5,266,331 to Oshlack et al, the teachings of which are incorporated herein by reference.

In another embodiment, the drug pellets and coated drug pellets are the venlafaxine spheroids disclosed in U.S. Patent No. 6,274,171 to Sherman et al., 6,403,120 to Sherman et al., and 6,419,958 to Sherman et al., the teachings of which are incorporated herein by reference.

In still another embodiment, the drug pellets and drug coated pellets are the tolterodine beads disclosed in U.S. Patent No. 6,630,162 to Nilvebrant, and 6,770,295 to Kreilgard et al., the teachings of which are incorporated herein by reference.

In one embodiment, the drug pellets and drug coated pellets are the opioid analgesic (e.g. hydromorphone) multiparticulates disclosed in U.S. Patent No. 5,958,452 to Oshlack et al., 5,965,161 to Oshlack et al., 5,968,551 to Oshlack et al., 6,294,195 to Oshlack et al., 6,335,033 to Oshlack et al., 6,706,281 to Oshlack et al., 6,743,442 to Oshlack et al., and 6,066,339 to Stark et al. the teachings of which are incorporated herein by reference.

In still another embodiment, the drug pellets and drug coated pellets are the amphetamine beads disclosed in U.S. Patent No. 6,322,819 and 6,605,300 both to Burnside et al., the teachings of which are incorporated herein by reference.

The drug pellets can be prepared by any known method in the art including melt pelletization techniques, extrusion with optional further shape modification of the resulting pellet, spheronization techniques, and the like.

The drug pellets can be coated to form coated drug pellets. The coating can be a functional or a non-functional coating, or multiple functional and/or non-functional coatings. By "functional coating" is meant to include a coating that modifies the release properties of the total formulation, for example, a sustained-release coating, extended-release coating, delayed-release coating, and the like. By "non-functional coating" is meant to include a coating that is not a functional coating, for example, a cosmetic coating. A non-functional coating can have some impact on the release of the active agent due to the initial dissolution, hydration, perforation of the coating, etc., but would not be considered to be a significant deviation from the non-coated form.

The coating material may include a polymer, for example, alkyl cellulose (e.g., methyl cellulose, ethyl cellulose, and the like), hydroxypropyl cellulose, hydroxypropyl methyl cellulose, hydroxybutyl methyl cellulose, cellulose acetate, cellulose propionate, cellulose acetate propionate, cellulose acetate butyrate, cellulose acetate phthalate, carboxymethyl cellulose, cellulose triacetate, cellulose sulphate sodium salt, poly(methyl methacrylate), poly (ethyl methacrylate), poly (butyl methacrylate), poly (isobutyl methacrylate), poly (hexyl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene)high density, (poly propylene), poly (ethylene glycol), poly (ethylene oxide), poly (ethylene terephthalate), poly(vinyl alcohol), poly(vinyl isobutyl ether), poly(vinyl acetate), poly (vinyl chloride), polyvinyl pyrrolidone, and combinations thereof.

The coating may also contain an effective amount of a plasticizer in the coating composition to improve the physical properties of the film. For example, because ethyl cellulose has a relatively high glass transition temperature and does not form flexible films under normal coating conditions, it may be advantageous to add plasticizer to the ethyl cellulose before using the same as a coating material. Generally, the amount of plasticizer included in a coating solution is based on the concentration of the polymer, e.g., most often from about 1 weight percent to about 50 weight percent of the polymer. Concentrations of the plasticizer, however, can be determined by routine experimentation.

Exemplary plasticizers for ethyl cellulose and other celluloses include dibutyl sebacate, diethyl phthalate, triethyl citrate, tributyl citrate, triacetin, and combinations thereof, although it is possible that other water-insoluble plasticizers (such as acetylated monoglycerides, phthalate esters, castor oil, etc.) can be used.

Exemplary plasticizers for acrylic polymers include citric acid esters such as triethyl citrate NF, tributyl citrate, dibutyl phthalate, 1,2-propylene glycol, polyethylene glycols, propylene glycol, diethyl phthalate, castor oil, triacetin, and combinations thereof, although it is possible that other plasticizers (such as acetylated monoglycerides, phthalate esters, castor oil, etc.) can be used.

An example of a functional coating comprises a coating agent comprising a poorly-water-permeable component (a) such as, an alkyl cellulose, for example an ethylcellulose, such as AQUACOAT (a 30% dispersion available from FMC, Philadelphia, PA) or SURELEASE (a 25% dispersion available from Colorcon, West Point, PA) and a water-soluble component (b), e.g., an agent that can form channels through the poorly-water-permeable component upon the hydration or dissolution of the soluble component. Specifically, the water-soluble component is a low molecular weight, polymeric material, e.g., a hydroxyalkylcellulose, hydroxyalkyl(alkylcellulose), and carboxymethylcellulose, or salts thereof. Particular examples of these water soluble polymeric materials include hydroxyethylcellulose, hydroxypropylcellulose, hydroxyethylmethylcellulose, hydroxypropylmethylcellulose, carboxymethylcellulose, sodium carboxymethylcellulose, and combinations comprising one or more of the foregoing materials. The water-soluble component can comprise hydroxypropylmethylcellulose, such as METHOCEL (Dow). The water-soluble component is of relatively low molecular weight, specifically less than or equal to about 25,000 molecular weight, or specifically less than or equal to about 21,000 molecular weight.

In the functional coating, the total of the water soluble portion (b) and poorly-water permeable portion (a) are present in weight ratios (b):(a) of about 1:4 to about 2:1, specifically about 1:2 to about 1:1, and more specifically in a ratio of about 2:3. Other ratios can be used to modify the speed with which the coating permits release of the active agent.

Exemplary delayed-release coatings include enteric coatings prepared from enteric polymers. The enteric polymer should be non-toxic and is predominantly soluble in the intestinal fluid, but substantially insoluble in the gastric juices. Examples include polyvinyl acetate phthalate (PVAP), hydroxypropylmethyl-cellulose acetate succinate (HPMCAS), cellulose acetate phthalate (CAP), methacrylic acid copolymer, hydroxy propyl methylcellulose succinate, cellulose acetate succinate, cellulose acetate hexahydrophthalate, hydroxypropyl methylcellulose hexahydrophthalate, hydroxypropyl methylcellulose phthalate (HPMCP), cellulose propionate phthalate, cellulose acetate maleate, cellulose acetate trimellitate, cellulose acetate butyrate, cellulose acetate propionate, methacrylic acid/methacrylate polymer (acid number 300 to 330 and also known as EUDRAGIT L), which is an anionic copolymer based on methacrylate and available as a powder (also known as methacrylic acid copolymer, type A NF, methacrylic acid-methyl methacrylate copolymer, ethyl methacrylate-methylmethacrylate-chlorotrimethylammonium ethyl methacrylate copolymer, and the like, and combinations comprising one or more of the foregoing enteric polymers. Other examples include natural resins, such as shellac, SANDARAC, copal collophorium, and combinations comprising one or more of the foregoing polymers. Yet other examples of enteric polymers include synthetic resin bearing carboxyl groups. The methacrylic acid: acrylic acid ethyl ester 1:1 copolymer solid substance of the acrylic dispersion sold under the trade designation "EUDRAGIT L-100-55" may be suitable.

Suitable methods known in the art can be used to apply the coating to the drug pellet. Processes such as simple or complex coacervation, interfacial polymerization, liquid drying, thermal and ionic gelation, spray drying, spray chilling, fluidized bed coating, pan coating, electrostatic deposition, may be used. A substantially continuous nature of the coating may be achieved, for example, by spray drying from a suspension or dispersion of the drug pellets in a solution of the coating composition including a polymer in a solvent in a drying gas having a low dew point.

When a solvent is used to apply the coating, the solvent is specifically an organic solvent that constitutes a good solvent for the coating material, but is substantially a non-solvent or poor solvent for of the drug pellet. The solvent may be selected from alcohols such as methanol, ethanol, halogenated hydrocarbons such as dichloromethane (methylene chloride), hydrocarbons such as cyclohexane, and combinations comprising one or more of the foregoing solvents. Dichloromethane (methylene chloride) has been found to be particularly suitable.

The functional coating may comprise about 1 weight percent to about 40 weight percent, specifically about 3 weight percent to about 30 weight percent, more specifically about 5 weight percent to about 25 weight percent, and yet more specifically about 6 weight percent to about 10 weight percent of the total pellet weight.

The drug pellets and drug coated pellets are not limited in size. The drug pellets or drug coated pellets can have an average diameter of about 100 micrometers or greater, specifically about 100 to about 3000 micrometers, more specifically about 500 to about 1800 micrometers, and yet more specifically about 700 to about 1200 micrometers.

The amount of coated drug pellet in the compressed pharmaceutical composition can be up to about 70 percent by weight based on the total tablet. When the integrity of the pellet and pellet coating is desired, lower amounts are suggested due to the potential damage caused by pellet to pellet interaction at high loadings. Specifically the amount of coated drug pellet can be about 10 to about 60 weight percent, more specifically about 20 to about 50 weight percent, and yet more specifically about 30 to about 40 weight percent based on the total weight of the compressed pharmaceutical composition.

Another aspect is to provide a process to make compressed pharmaceutical compositions containing coated drug pellets. The compressible mixtures as disclosed herein, are particularly suited for direct compression processes even with drug pellets coated with material that is considered inelastic and easily subject to damage when compressed (e.g. ethyl cellulose). Direct compression, using commercially available punches and dies fitted to a suitable rotary tableting press, can be used. In common tableting processes, the material that is to be tableted is deposited into a cavity, and one or more punch members are then advanced into the cavity and brought into intimate contact with the material to be pressed, whereupon compressive force is applied. The material is thus forced into conformity with the shape of the punches and the cavity.

The compression force that is suitable for preparing the compressed pharmaceutical compositions via direct compression can be about 2 to about 25 kiloNewtons (kN), specifically about 3 to about 20 kN, and more specifically about 5 to about 15 kN. It has been found that within these ranges, the tableting force used has little effect on the dissolution profile of the resulting compressed pharmaceutical composition.

The compressed pharmaceutical compositions formed exhibit a hardness of at least about 5 kilopond (kp), specifically at least about 8 kp; more specifically at least about 10 kp; and yet more specifically at least about 12 kp. Direct compression techniques are preferred for the formation of the compressed pharmaceutical compositions. When compressed pharmaceutical compositions are made by direct compression, the addition of lubricants may be helpful to promote powder flow and to prevent capping of the particle (breaking off of a portion of the particle) when the pressure is relieved.

The compressed pharmaceutical compositions do not have to undergo post-compression processes such as sintering or coating as the compressed compositions exhibit suitable hardness and friability characteristics. However, if desired the tablets may optionally be further coated with a non-functional or functional coating as described above. Additionally, the compressed compositions can undergo a sintering process to meld the surface of the composition.

The compressed pharmaceutical composition can be prepared in a variety of geometrical shapes and sizes by use of different punches and dies. The compressed pharmaceutical composition can be compressed into scored forms (e.g. one score line to allow for the tablet to be split into two; or two score lines to allow the tablet to be split into three pieces) or, as discussed previously, compressed compositions free of a score.

In one embodiment, a compressed pharmaceutical composition is formed by the process comprising mixing a powdered non-water soluble waxy filler and a disintegrant to form a compressible mixture having average particle diameters of about 0.1 to about 125 micrometers, with the proviso that the compressible mixture is not prepared by extrusion, spheronization, high shear mixing, melt blending, melt pelletization, freeze-drying, or a combination thereof; mixing the compressible mixture with a plurality of coated pellets to form a compressible mixture, wherein the coated pellets comprise a pharmaceutically active agent or salt, solvate, hydrate, or polymorph thereof; and directly compressing the compressible mixture into a compressed pharmaceutical composition.

The drug pellets, coated drug pellets, and the compressed pharmaceutical compositions prepared from compressing a mixture of pellets and compressible mixture can be characterized by their dissolution profiles. Dissolution profile as used herein, means a plot of the cumulative amount of active ingredient released as a function of time. The dissolution profile can be measured utilizing the Drug Release Test <724>, which incorporates standard test USP 28 (Test <711>). A profile is characterized by the test conditions selected. Thus the dissolution profile can be generated at a pre-selected apparatus type, shaft speed, temperature, volume, and pH of the dissolution media.

A first dissolution profile can be measured at a pH level approximating that of the stomach. A second dissolution profile can be measured at a pH level approximating that of one point in the intestine or several pH levels approximating multiple points in the intestine.

A highly acidic pH may simulate the stomach and a less acidic to basic pH may simulate the intestine. By the term "highly acidic pH": it is meant a pH of about 1 to about 4. By the term "less acidic to basic pH" is meant a pH of greater than about 4 to about 7.5, specifically about 6 to about 7.5. A pH of about 1.2 can be used to simulate the pH of the stomach. A pH of about 6 to about 7.5, specifically about 6.8, can be used to simulate the pH of the intestine. Exemplary dissolution media include 500 or 900 ml of purified water; an aqueous buffer solution (USP, pH 4.5); an aqueous buffer solution (USP, pH 6.8); an aqueous buffer solution (USP, pH 7.5); or 0.1N HCl.

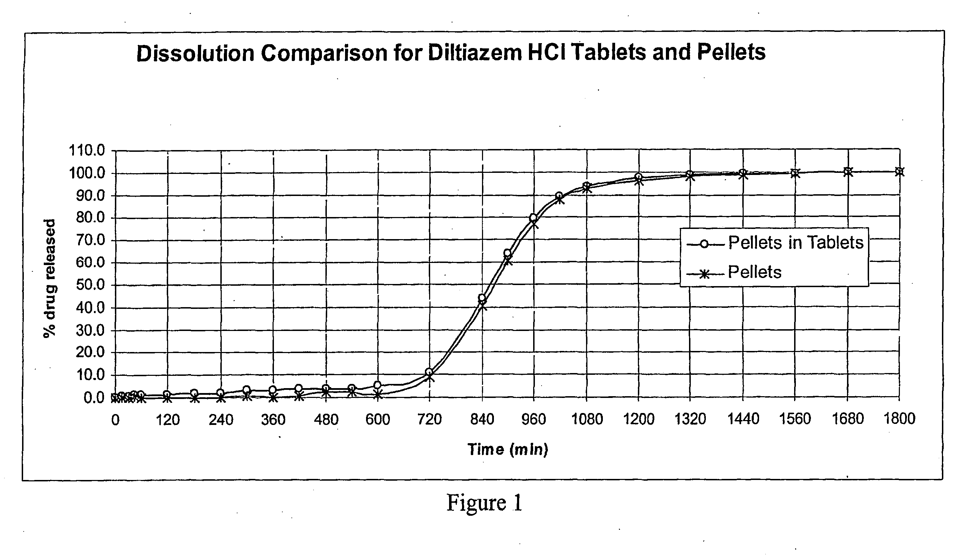

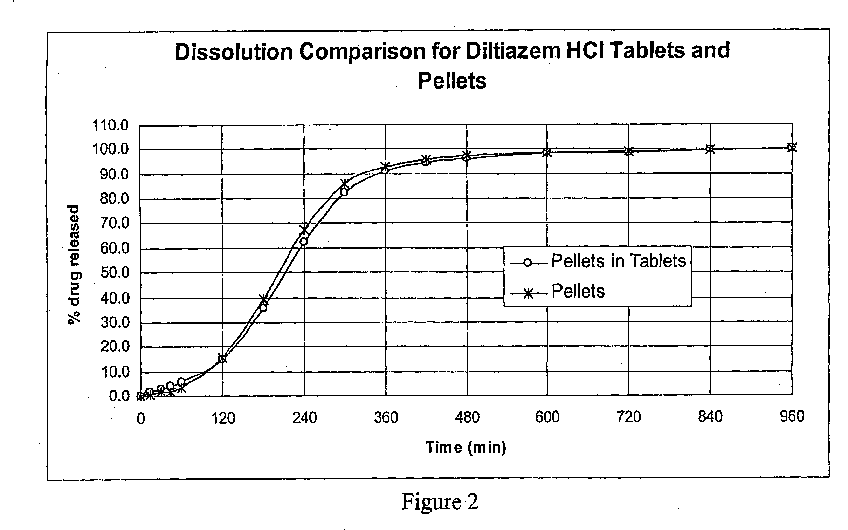

In one embodiment, the dissolution profile exhibited by the compressed pharmaceutical composition prepared by compressing a blend of compressible mixture and coated drug pellets is substantially the same as the dissolution profile of the coated drug pellets alone. As used herein, "substantially the same dissolution profile" means that the dissolution rate of the compressed pharmaceutical composition varies from the dissolution rate of the coated pellets by less than or equal to about 5 percent as determined at any point in the sharpest slope of the dissolution profile (e.g. between 720 and 1020 minutes in Figure 1 and between 120 and 300 minutes in Figure 2). The dissolution profile is substantially the same between the tablet and the coated pellets when tested according to an USP compendia method (e.g., 500 or 900 ml of purified water, USP aqueous buffer at pH 4.5, USP aqueous buffer at pH 6.8, USP aqueous buffer at pH 7.5, or 0.1N HCl at 37°C in Apparatus 2 (USP 28, < 711 > Dissolution, paddle, 50 rpm or 100 rpm paddle speed).

In one embodiment, the difference between the dissolution rate of the compressed pharmaceutical composition varies from the dissolution rate of the coated pellets by less than or equal to about 12 percent wherein the difference is determined at any point in the sharpest slope of the dissolution profile (e.g. between 720 and 1020 minutes in Figure 1 and between 120 and 300 minutes in Figure 2), specifically less than or equal to about 10 percent, more specifically less than or equal to about 8 percent, and yet more specifically less than or equal to about 5 percent.

The drug pellets, coated drug pellets, and the compressed pharmaceutical compositions prepared from compressing a blend of pellets and compressible mixture can be characterized by their pharmacokinetic parameters. "Pharmacokinetic parameters" are parameters which describe the *in vivo* characteristics of the active agent over time, including for example the *in vivo* dissolution characteristics and plasma concentration of the active agent. By "C*ₘₐₓ*" is meant the measured concentration of the active agent in the plasma at the point of maximum concentration. By "C*₂₄*" is meant the concentration of the active agent in the plasma at about 24 hours. The term "T*ₘₐₓ*" refers to the time at which the concentration of the active agent in the plasma is the highest. "AUC" is the area under the curve of a graph of the concentration of the active agent (typically plasma concentration) vs. time, measured from one time to another.

In one embodiment, a compressed pharmaceutical composition (e.g. tablet) prepared by compressing a blend of compressible mixture and coated drug pellets, after oral administration thereof to a mammal, exhibits substantially the same bioavailability of the pharmaceutically active agent as the bioavailability of the pharmaceutically active agent achieved by the oral administration of the coated pellets in the absence of the compressible mixture. As disclosed herein, "substantially the same biovavailability" means that the bioavailability of the compressed pharmaceutical compositions exhibits an AUC, Tₘₐₓ, and Cₘₐₓ that varies by less than or equal to about 5 percent as compared to the coated pellets alone.

In one embodiment, a compressed pharmaceutical composition (e.g. tablet) prepared by compressing a blend of compressible mixture and coated drug pellets, exhibits an AUC, Tₘₐₓ, and Cₘₐₓ after oral administration thereof to a mammal that varies by less than or equal to about 15 percent from a corresponding dose of coated drug pellets dosed orally to a mammal in the absence of the compressible mixture. More specifically the AUC, Tₘₐₓ, and Cₘₐₓ of the compressed pharmaceutical composition varies by less than or equal to about 10 percent, yet more specifically less than or equal to about 7.5 percent.

In one embodiment, the compressible mixture is free of paraffin wax, microcrystalline wax, and/or microcrystalline cellulose.

The compressed pharmaceutical compositions provided herein can be formulated to be easily broken by hand using finger pressure without the presence of a score line. The compressed pharmaceutical composition can be broken into substantially uniform pieces without the aid of a score line. As used herein, "substantially uniform pieces" means that the weight of each piece is within about 10 percent of one another.

All references, including publications, patent applications, and patents, cited herein are hereby incorporated by reference to the same extent as if each reference were individually and specifically indicated to be incorporated by reference and were set forth in its entirety herein.

The following examples further illustrate the invention but, of course, should not be construed as in any way limiting its scope.

### EXAMPLE 1

### Preparation of a diltiazem extended-release pellet:

Diltiazem extended-release pellets were prepared by coating 500 micrometer sugar spheres with diltiazem hydrochloride and hydroxypropyl cellulose to form a drug coated pellet. The drug coated pellet was then coated with a controlled-release coating, which was a combination of Eudragit RS and Eudragit RL in a 93.4 : 6.6 ratio. This coating contributes to about 20% of the pellet weight. The average diameter size of the coated diltiazem pellets was about 900 micrometers.

### Preparation of diltiazem tablets containing a carnauba wax/cross-linked sodium carboxymethylcellulose compressible mixture and diltiazem extended-release pellets:

The compressible mixture used to prepare the tablets include commercial grade powdered Carnauba wax and Ac-Di-Sol® available from FMC BioPolymer of Philadelphia, PA as the main components. The tablets were prepared by mixing all of the components of Table 1 except the magnesium stearate to form a uniform mixture followed by the addition of the magnesium stearate with mixing to form a fmal mixture. The final mixture was compressed into direct compression tablets using a Kilian LX Tablet press with an 0.75 x 0.45 inch oval-shaped tooling. Tablets were prepared for each of the following compression forces: 3, 4, 8, 10, and 14 kiloNewtons (kN), to produce tablets exhibiting a hardness of 4, 6, 7, 7, and 11 kiloponds (kp) respectively. No segregation was observed for the coated pellets and the compressible mixture. Such a result was especially surprising in view of the large size of the diltiazem pellets as compared to the remaining components which are powders.

**Table 1.**

| **mg/tablet** | **Ingredients** | **gram/batch** |
|---|---|---|
| 400 | Diltiazem hydrochloride Pellets | 400 |
| 570 | Carnauba Wax (powder) | 570 |
| 20 | AC-DI-SOL | 20 |
| 10 | Mg Stearate | 10 |
| 1000 | Total wt. | 1000 |

The compressed tablets and the diltiazem extended-release pellets were tested for dissolution according to USP 28 apparatus II using 900 milliliters 0.1 N HCl as the dissolution media at 37°C± 0.5°C using a paddle speed of 100 rotations per minute (rpm). UV spectrometer was used to determine the amount of diltiazem dissolved at a given time point. The dissolution results are provided in Table 2 below as percentage of diltiazem released. The data is also illustrated graphically in Figure 1. As can be seen from the comparison of the dissolution results, the compressed tablets exhibit a dissolution profile substantially identical to the free pellets. Such a result exhibits both the ability of the compressible mixture to protect the pellet coating while at the same time not delaying the dissolution of the diltiazem from the tablet. It is difficult to achieve a zero percent release as there will always be some minimal damage to the pellet or pellet coating for those pellets at the interface with the tableting tools. As indicated by the dissolution profiles in the Figure, such damage is minimized by the use of the compressible mixture.

**Table 2**

| | % Drug Released | |
|---|---|---|
| Time (minutes) | Pellets in Tablets | Pellets |
| 0 | 0.0 | 0.0 |
| 15 | 0.7 | 0.1 |
| 30 | 0.9 | 0.1 |
| 45 | 1.3 | 0.5 |
| 60 | 1.0 | 0.2 |
| 120 | 1.5 | 0.2 |
| 180 | 1.9 | 0.2 |
| 240 | 2.2 | 0.1 |
| 300 | 3.0 | 0.3 |
| 360 | 3.2 | 0.0 |
| 420 | 3.6 | 0.4 |
| 480 | 3.9 | 2.3 |
| 540 | 3.9 | 2.3 |
| 600 | 4.8 | 1.5 |
| 720 | 10.8 | 8.6 |
| 840 | 43.5 | 40.6 |
| 900 | 63.8 | 60.6 |
| 960 | 79.7 | 76.9 |
| 1020 | 89.1 | 87.9 |
| 1080 | 93.7 | 93.0 |
| 1200 | 97.3 | 96.4 |
| 1320 | 98.5 | 97.9 |
| 1440 | 99.2 | 98.7 |
| 1560 | 99.1 | 99.3 |
| 1680 | 100.0 | 99.7 |
| 1800 | 100.0 | 100.0 |

### EXAMPLE 2

### Preparation of an additional example of diltiazem extended-release pellet:

Diltiazem extended-release pellets were prepared according to Example 1, but the controlled-release coating composition contributes to about 10% of the pellet weight. The average diameter size of the coated diltiazem pellets was about 850 micrometers.

Preparation of diltiazem tablets containing a carnauba wax/cross-linked sodium carboxymethylcellulose compressible mixture and diltiazem extended-release pellets:

The tablets were prepared with the components of Table 3, according to the procedure of Example 1. Tablets were prepared for each of the following compression forces: 4, 8, 12, and 21 kiloNewtons (kN), to produce tablets exhibiting a hardness of 7, 8, 10, and 10 kiloponds (kp) respectively. No segregation was observed for the coated pellets and the compressible mixture.

**Table 3.**

| **mg/tablet** | **Ingredients** | **gram/batch** |
|---|---|---|
| 400 | Diltiazem hydrocloride Pellets | 400 |
| 570 | Carnauba Wax (powder) | 570 |
| 20 | AC-DI-SOL | 20 |
| 10 | Mg Stearate | 10 |
| 1000 | Total wt. | 1000 |

The compressed tablets and the diltiazem extended-release pellets were tested for dissolution according to the procedure of Example 1. The results of the dissolution analysis is provided in Table 4 below and illustrated graphically in Figure 2. As can be seen from the data, the dissolution profile of the tablets prepared from the coated pellets and compressible mixture is substantially the same as the coated pellets themselves.

**Table 4.**

| | **% Drug Released** | |
|---|---|---|
| **Time** | **Pellets in Tablets** | **Pellets** |
| 0 | 0.0 | 0.0 |
| 15 | 1.5 | 0.8 |
| 30 | 2.8 | 1.5 |
| 45 | 3.8 | 1.9 |
| 60 | 5.6 | 3.5 |
| 120 | 14.9 | 15.8 |
| 180 | 36.0 | 39.4 |
| 240 | 62.4 | 67.4 |
| 300 | 82.3 | 85.8 |
| 360 | 90.9 | 92.8 |
| 420 | 94.6 | 95.6 |
| 480 | 96.3 | 97.4 |
| 600 | 98.2 | 98.6 |
| 720 | 98.4 | 98.8 |
| 840 | 99.4 | 99.4 |
| 960 | 100.0 | 100.0 |

### EXAMPLE 3

### Preparation of a morphine extended-release pellet:

Morphine extended-release pellets were prepared by coating a drug core containing morphine sulfate and hydroxypropyl methylcellulose to form a drug coated pellet. The drug coated pellet was coated with Eudragit L100 and ethylcellulose in a ratio of 77:23. The average diameter size of the morphine extended-release pellets was approximately 1400 micrometers.

Preparation of morphine extended-release tablets containing a carnauba wax/cross-linked sodium carboxymethylcellulose compressible mixture and morphine extended-release pellets:

The tablets were prepared with the components of Table 5, according to the procedure of Example 1. No segregation was observed for the coated pellets and the compressible mixture.

**Table 5.**

| **mg/tablet** | **Ingredients** | **gram/batch** |
|---|---|---|
| 400 | Morphine sulfate Pellets | 400 |
| 570 | Carnauba Wax (powder) | 570 |
| 20 | AC-DI-SOL | 20 |
| 10 | Mg Stearate | 10 |
| 1000 | Total wt. | 1000 |

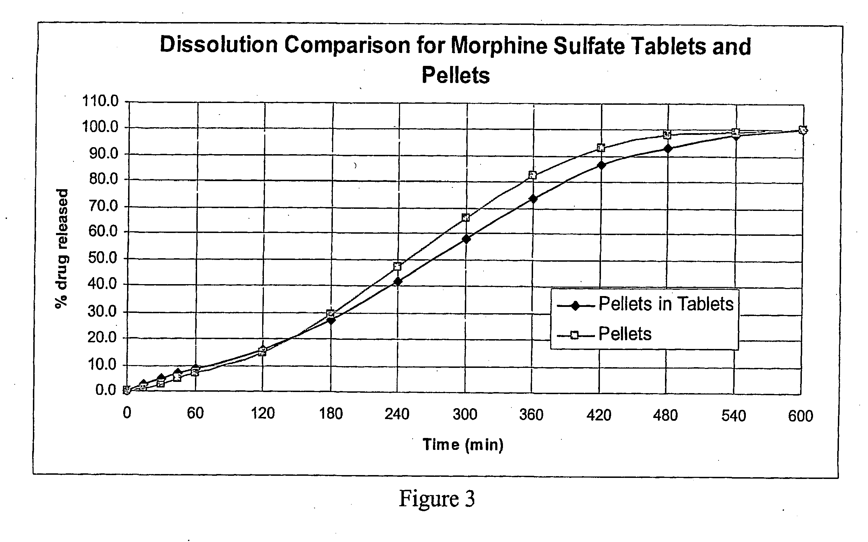

The compressed tablets and the morphine extended-release pellets were tested for dissolution according to the procedure of Example 1, but the dissolution medium used was 900 ml 0.1 N HCl for two (2) hours and then in pH 6.8 phosphate buffer for eight (8) hours. The results of the dissolution analysis is provided in Table 6 below and illustrated graphically in Figure 3. As can be seen from the data, the dissolution profile of the tablets prepared from the coated pellets and compressible mixture is slightly slower as compared to the coated pellets alone, but with the same overall endpoint. As indicated, the result was minimal damage to the pellets or the coatings on the pellets and therefore no increase in the dissolution.

**Table 6.**

| | **% Drug Released** | |
|---|---|---|
| **Time** | **Pellets in Tablets** | **Pellets** |
| 0 | 0.0 | 0.0 |
| 15 | 2.8 | 1.2 |
| 30 | 4.6 | 2.6 |
| 45 | 7.1 | 4.9 |
| 60 | 8.9 | 6.8 |
| 120 | 15.9 | 14.8 |
| 180 | 27.0 | 29.0 |
| 240 | 41.7 | 47.2 |
| 300 | 57.9 | 66.1 |
| 360 | 73.5 | 82.5 |
| 420 | 86.8 | 93.3 |
| 480 | 93.0 | 97.9 |
| 540 | 97.9 | 99.3 |
| 600 | 100.0 | 100.0 |

### EXAMPLE 4

### Preparation of an amphetamine extended-release pellet:

Amphetamine extended-release pellets were prepared using a combination of Eudragit RS and Eudragit RL in a 90:10 ratio. The resulting extended-release amphetamine pellets had an average diameter size of 640 micrometers.

Preparation of amphetamine extended-release tablets containing a carnauba wax/cross-linked sodium carboxymethylcellulose compressible mixture and amphetamine extended-release pellets:

The tablets were prepared with the components of Table 7, according to the procedure of Example 1 above. No segregation was observed for the coated pellets and the compressible mixture.

**Table 7.**

| **mg/tablet** | **Ingredients** | **gram/batch** |
|---|---|---|
| 400 | Amphetamine Pellets | 400 |
| 570 | Carnauba Wax (powder) | 570 |
| 20 | AC-DI-SOL | 20 |
| 10 | Mg Stearate | 10 |
| 1000 | Total wt. | 1000 |

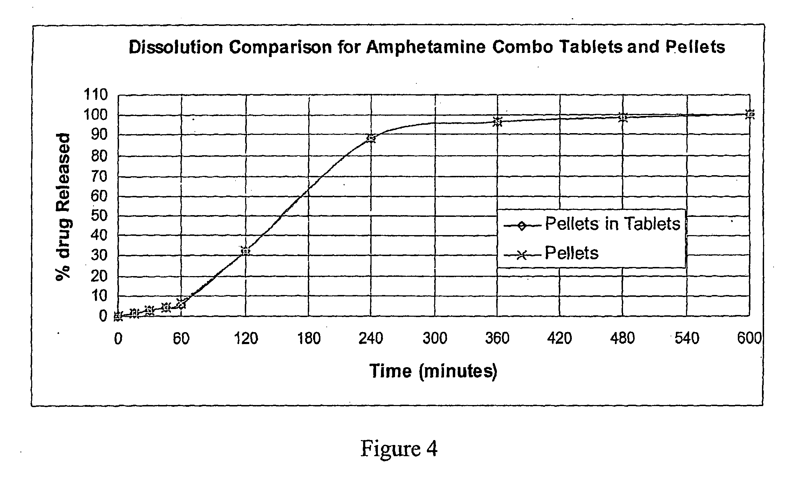

The compressed tablets and the amphetamine extended-release pellets were tested for dissolution according to the procedure of Example **1**. The dissolution samples were analyzed by HPLC. The results of the dissolution analysis is provided in Table 8 below and illustrated graphically in Figure 4. As can be seen from the data, the dissolution profile of the tablets prepared from the coated pellets and compressible mixture is substantially the same as the coated pellets themselves.

**Table 8.**

| | **% Drug Released** | |
|---|---|---|
| **Time** | **Pellets in Tablets** | **Pellets** |
| 0 | 0.0 | 0.0 |
| 15.0 | 1.1 | 1.1 |
| 30.0 | 2.6 | 2.8 |
| 45.0 | 4.1 | 4.2 |
| 60.0 | 5.7 | 6.1 |
| 120.0 | 32.4 | 32.7 |
| 240.0 | 88.1 | 88.0 |
| 360.0 | 96.5 | 96.4 |
| 480.0 | 98.9 | 98.6 |
| 600.0 | 100.0 | 100.0 |

### Example 5.

### Preparation of diltiazem tablets containing a carnauba wax/microcrystalline cellulose/cross-linked sodium carboxymethylcellulose compressible mixture and diltiazem extended-release pellets:

The materials used to prepare the tablets include commercial grade Avicel PH102, Carnauba wax and Ac-Di-Sol® available from FMC BioPolymer of Philadelphia, PA. The tablets were prepared by mixing all of the components of Table 9 except the magnesium stearate to form a uniform mixture followed by the addition of the magnesium stearate with mixing to form a final mixture. The final mixture was compressed into direct compression tablets using a Kilian LX Tablet press with an 0.497 x 0.272 inch oval-shaped tooling. Tablets were prepared for each of the following compression forces: 8 and 10 kN, to produce tablets exhibiting a hardness of about 8 to 12 kp, respectively. No segregation was observed for the coated pellets and the compressible mixture.

**Table 9.**

| **mg/Tablet** | **Ingredients** | **gram/batch** |
|---|---|---|
| 133 | Diltiazem Pellets | 400 |
| 157 | Carnauba Wax (powder) | 470 |
| 33 | Avicel PH102 | 100 |
| 7 | AC-DI-Sil | 20 |
| 3 | Mg Stearate | 10 |
| 333 | Total wt. | 1000 |

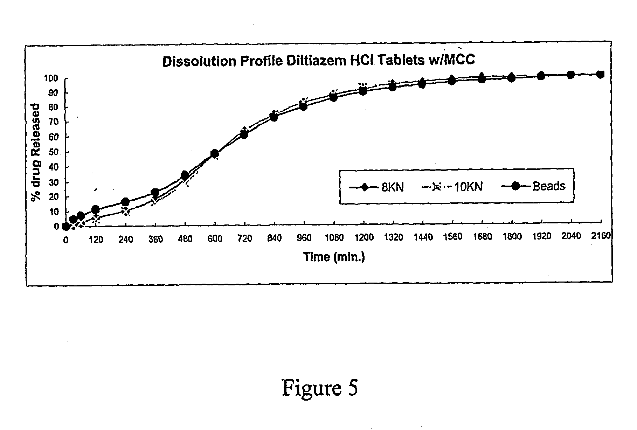

The compressed tablets for both compression forces and the diltiazem extended-release pellets were tested for dissolution according to USP 28 apparatus II using 900 milliliters 0.1 N HCl as the dissolution media at 37°C± 0.5°C using a paddle speed of 100 rotations per minute (rpm). Analysis of the dissolution samples were performed according to Example 1. The dissolution results are provided in Table 10 below as percentage of diltiazem released. The data is also illustrated graphically in Figure 5. As can be seen from the comparison of the dissolution results, both of the compressed tablets exhibit a dissolution profile substantially identical to the free pellets. The particular combination of the carnauba wax powder, microcrystalline cellulose and disintegrant provides a compressible mixture capable of forming strong tablets (as evidenced by the hardness results) by compression while at the same time providing enough protection for the coated beads to maintain the integrity of the coating (as evidenced by the dissolution results). Furthermore, no segregation of the compressible mixture from the pellets was observed during the tableting process.

**Table 10.**

| | % Drug Released | | |
|---|---|---|---|
| Time (minutes) | Pellets in Tablets 8 KN | Pellets in Tablets 10 KN | Pellets |
| 0 | 0.0 | 0.0 | 0.0 |
| 30 | 0.8 | 1.1 | 4.7 |
| 60 | 2.0 | 2.5 | 7.3 |
| 120 | 5.6 | 4.8 | 11.1 |
| 240 | 10.1 | 9.7 | 16.1 |
| 360 | 18.0 | 16.1 | 22.6 |
| 480 | 31.1 | 28.8 | 33.8 |
| 600 | 47.6 | 46.6 | 48.0 |
| 720 | 63.0 | 62.7 | 60.6 |
| 840 | 73.7 | 73.7 | 71.9 |
| 960 | 82.3 | 82.5 | 78.8 |
| 1080 | 87.6 | 88.2 | 85.4 |
| 1200 | 91.7 | 92.2 | 89.5 |
| 1320 | 94.4 | 93.9 | 92.1 |
| 1440 | 95.9 | 95.7 | 94.3 |
| 1560 | 97.3 | 97.0 | 95.9 |
| 1680 | 98.9 | 98.1 | 97.1 |
| 1800 | 98.9 | 98.7 | 98.0 |
| 1920 | 99.6 | 99.6 | 99.0 |
| 2040 | 100 | 99.8 | 99.8 |
| 2160 | 100 | 100 | 100 |

## Claims

1. A compressed pharmaceutical composition, comprising:
a plurality of coated pellets, wherein the coated pellets comprise a pharmaceutically active agent or salt, solvate, hydrate, or polymorph thereof; and
a compressible mixture, wherein the compressible mixture comprises i) a non-water soluble waxy filler and ii) a disintegrant, wherein the weight ratio of waxy filler to disintegrant is 15:1 to 50:1 and wherein the compressible mixture is in powder form with an average particle diameter of 0.1 to 175 micrometers,
with the proviso that the compressible mixture is not prepared by extrusion, spheronization, high shear mixing, melt blending, melt pelletization, freeze-drying, or a combination thereof;
wherein the compressed composition exhibits an in vitro dissolution profile according to a USP compendia method that is substantially the same as the dissolution of the coated pellets in the absence of the compressible mixture.

2. The compressed pharmaceutical composition of claim 1, wherein, after oral administration thereof to a mammal, the bioavailability of the pharmaceutically active agent from the compressed composition is substantially the same as the bioavailability of the pharmaceutically active agent achieved by the administration of the coated pellets in the absence of the compressible mixture.

3. The compressed pharmaceutical composition of claim 1, wherein the USP compendia method is USP 28, < 711 > Dissolution, Apparatus 2, paddle, paddle speed of 100 rpm and 900 ml of 0.1 N HCl as a dissolution medium at 37°C.

4. The compressed pharmaceutical composition of claim 1, wherein the waxy filler is carnauba wax, vegetable wax, fruit wax, microcrystalline wax, bees wax, hydrocarbon wax, paraffin wax, cetyl esters wax, non-ionic emulsifying wax, anionic emulsifying wax, candelilla wax, stearyl alcohol, cetyl alcohol, cetostearyl alcohol, polyethylene glycol having a Mn of greater than 3000, or combinations thereof; and
the disintegrant is cross-linked sodium carboxymethylcellulose, crosslinked homopolymer of N-vinyl-2-pyrrolidone, modified starches, sodium carboxymethyl starch, sodium starch glycolate, alginates, starch, pregelatinized starch, or combinations thereof.

5. The compressed pharmaceutical composition of claim 1, wherein the waxy filler is powdered carnauba wax and the disintegrant is cross-linked sodium carboxymethylcellulose.

6. The compressed pharmaceutical composition of claim 1, wherein the amount of waxy filler is 25 to 75 weight percent based on the total weight of the compressed pharmaceutical composition.

7. The compressed pharmaceutical composition of claim 1, wherein the amount of disintegrant is 1 to 5 weight percent based on the total weight of the compressed pharmaceutical composition.

8. The compressed pharmaceutical composition of claim 1, further comprising an additional excipient, an additional disintegrant, a coating disposed on the surface of the compressed composition, or combinations thereof.

9. The compressed pharmaceutical composition of claim 8, wherein the additional excipient is a binder, a lubricant, a glidant, a compression aid, a colorant, a preservative, a flavor, or combinations thereof.

10. The compressed pharmaceutical composition of claim 1, wherein the compressible mixture further comprises a cellulose filler.

11. The compressed pharmaceutical composition of claim 1, wherein the compressible mixture further comprises microcrystalline cellulose in a weight ratio to the waxy filler of 5:95 to 40:60 (w/w).

12. The compressed pharmaceutical composition of claim 1, wherein the pharmaceutically active agent or salt thereof is an alpha-2 adrenergic agent, an analgesic, an angiotensin-converting enzyme (ACE) inhibitor, an antianxiety agent, an antiarrhythmic, an antibacterial, an antibiotic, an antidepressant, an antidiabetic, an antiemetic, an antiepileptic, an antifungal, an antihelminthic, an antihistamine, an antihyperlipidemic, an antihypertensive agent, an antiinfective, an antimalarial, an antimicrobial, an antimigraine agent, an antimuscarinic agent, an antineoplastic agent, an antiprotozoal agent, an antipsychotic agent, an antispasmodic, an antiviral agent, an attention-deficit hyperactivity disorder (ADHD) agent, a β-blocker, a calcium channel blocker, a chemotherapeutic agent, a cholinesterase inhibitor, a Cox-2 inhibitor, a decongestant, a diuretic, a histamine-2 receptor antagonist, a hypnotic, a hypotensive agent, an immunosuppressant, a lipotropic, a neuroleptic, an opioid analgesic, a peripheral vasodilator/vasoconstrictor, a sedative, a serotonin receptor agonist, or pharmaceutically acceptable combinations thereof.

13. The compressed pharmaceutical composition of claim 1, wherein the pharmaceutically active agent is amphetamine, dextroamphetamine, diltiazem, fluvastatin, hydromorphone, morphine, oxybutynin, oxycodone, paroxetine, propranolol, tolterodine, venlafaxine, or the pharmaceutically acceptable salt, solvate, hydrate, or polymorph thereof.

14. The compressed pharmaceutical composition of claim 1, wherein the coated pellets comprise a coating prepared from alkyl cellulose, methyl cellulose, ethyl cellulose, hydroxypropyl cellulose, hydroxypropyl methyl cellulose, hydroxybutyl methyl cellulose, cellulose acetate, cellulose propionate, cellulose acetate propionate, cellulose acetate butyrate, cellulose acetate phthalate, carboxymethyl cellulose, cellulose triacetate, cellulose sulphate sodium salt, poly(methyl methacrylate), poly (ethyl methacrylate), poly (butyl methacrylate), poly (isobutyl methacrylate), poly (hexyl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, (poly propylene), poly (ethylene glycol), poly (ethylene oxide), poly (ethylene terephthalatc), poly(vinyl alcohol), poly(vinyl isobutyl ether), poly(vinyl acetate), poly (vinyl chloride), polyvinyl pyrrolidone, or combinations thereof; and optionally further comprising a plasticizer.

15. The compressed pharmaceutical composition of claim 1, wherein the coated pellets have an average diameter size of 100 to 3000 micrometers.

16. The compressed pharmaceutical composition of claim 1, wherein the compressible mixture comprises particles having an average diameter size of 0.1 to 125 micrometers.

17. The compressed pharmaceutical composition of claim 1, wherein the compressed pharmaceutical composition has a hardness value of at least 8 kiloponds.

18. The compressed pharmaceutical composition of claim 1, wherein the weight ratio of waxy filler to disintegrant is 20:1 to 45:1.

19. A process of making a compressed pharmaceutical composition, comprising:
mixing a powdered, waxy filler and a disintegrant to form a compressible mixture with an average particle diameter of 0.1 to 175 micrometers, wherein the waxy filler is non-water soluble and wherein the weight ratio of waxy filler to disintegrant is 15:1 to 50:1,
with the proviso that the compressible mixture is not prepared by extrusion, spheronization, high shear mixing, melt blending, melt pelletization, freeze-drying, or a combination thereof;
mixing the compressible mixture with a plurality of coated pellets to form a pellet mixture, wherein the coated pellets comprise a pharmaceutically active agent or salt, solvate, hydrate, or polymorph thereof; and
compressing the pellet mixture with conventional direct compression equipment, wherein the compression force is not more than about 25 kiloNewtons.

20. A direct compression compressible mixture for forming compressed pharmaceutical compositions, comprising:
a mixture of a powdered waxy filler and a disintegrant, wherein the weight ratio of waxy filler to disintegrant is 15:1 to 50:1, and optionally further comprising a cellulose filler, a binder, a lubricant, a glidant, a compression aid, a colorant, a preservative, a flavor, or combinations thereof,
wherein the mixture has an average particle diameter of 0.1 to 175 micrometers, with the proviso that the compressible mixture is not prepared by extrusion, spheronization, high shear mixing, melt blending, melt pelletization, freeze-drying, or a combination thereof;
wherein the compressible mixture is suitable for directly compressing mixtures of the compressible mixture and coated drug pellets with substantially no damage to the coated drug pellets.

21. The compressible mixture of claim 20, wherein the powdered waxy filler is carnauba wax, vegetable wax, fruit wax, microcrystalline wax, bees wax, hydrocarbon wax, paraffin wax, cetyl esters wax, non-ionic emulsifying wax, anionic emulsifying wax, candelilla wax, stearyl alcohol, cetyl alcohol, cetostearyl alcohol, polyethylene glycol having a Mn of greater than 3000, or combinations thereof; and
wherein the disintegrant is cross-linked sodium carboxymethylcellulose, crosslinked homopolymer of N-vinyl-2-pyrrolidone, modified starches, sodium carboxymethyl starch, sodium starch glycolate, alginates, starch, pregelatinized starch, and combinations thereof.

22. The compressible mixture of claim 20, wherein the compressible mixture is suitable for directly compressing mixtures of the compressible mixture and coated drug pellets to form a compressed pharmaceutical composition, wherein the dissolution profile exhibited by the compressed pharmaceutical composition is substantially the same as the dissolution profile of the coated drug pellets alone.

23. The compressible mixture of claim 22, wherein the powdered waxy filler is a non-water soluble powdered waxy filler.

24. The compressible mixture of claim 22, wherein the weight ratio of waxy filler to disintegrant is 20:1 1 to 45:1.

25. The compressible mixture of claim 22, wherein the weight ratio of waxy filler to disintegrant is 25:1 to 40:1.

26. The compressible mixture of claim 22, wherein the mixture of the powdered waxy filler and the disintegrant is in powder form.

27. The compressible mixture of claim 22, wherein the compressible mixture further comprises a cellulose filter.

28. The compressible mixture of claim 22, wherein the compressible mixture further comprises microcrystalline cellulose or powdered cellulose in a weight ratio to the waxy filler of 5:95 to 40:60 (w/w).

## Patentansprüche

1. Komprimierte pharmazeutische Zusammensetzung, umfassend:
eine Mehrzahl beschichteter Pellets, wobei die beschichteten Pellets einen pharmazeutischen Wirkstoff oder ein Salz, Solvat, Hydrat oder Polymorph davon umfassen; und
ein komprimierbares Gemisch, umfassend i) einen nicht-wasserlöslichen wachsartigen Füllstoff und ii) einen Zerfallsbeschleuniger, wobei das Gewichtsverhältnis des wachsartigen Füllstoffs zum Zerfallsbeschleuniger 15:1 bis 50:1 beträgt und wobei das komprimierbare Gemisch in Pulverform mit einem mittleren Partikeldurchmesser von 0,1 bis 175 µm vorliegt,
mit der Maßgabe, dass das komprimierbare Gemisch nicht durch Extrusion, Spheronisation, Mischen unter hohen Scherkräften, Schmelzvermischung, Schmelzpelletierung, Gefriertrocknung oder einer Kombination davon hergestellt ist;
wobei die komprimierte Zusammensetzung ein *in vitro* Freisetzungsprofil gemäß eines USP-Kompendienverfahrens aufweist, das im Wesentlichen das Gleiche ist wie bei der Freisetzung der beschichteten Pellets in Abwesenheit des komprimierbaren Gemisches.

2. Komprimierte pharmazeutische Zusammensetzung nach Anspruch 1, wobei die Bioverfügbarkeit des pharmazeutischen Wirkstoffs aus der komprimierten Zusammensetzung nach oraler Verabreichung an ein Säugetier im Wesentlichen die Gleiche ist wie die Bioverfügbarkeit des pharmazeutischen Wirkstoffs, die durch Verabreichung der beschichteten Pellets in Abwesenheit des komprimierbaren Gemisches erreicht wird.

3. Komprimierte pharmazeutische Zusammensetzung nach Anspruch 1, wobei es sich bei dem USP-Kompendienverfahren um USP 28, < 711 > Freisetzung, Apparat 2, Schaufelrad, Schaufelradgeschwindigkeit von 100 U/min und 900 mL einer 0,1 N HCl als Freisetzungsmedium bei 37°C handelt.

4. Komprimierte pharmazeutische Zusammensetzung nach Anspruch 1, wobei es sich bei dem wachsartigen Füllstoff um Carnaubawachs, pflanzliches Wachs, Fruchtwachs, mikrokristallines Wachs, Bienenwachs, Kohlenwasserstoffwachs, Parraffinwachs, Cetylesterwachs, nicht-ionisch emulgierendes Wachs, anionisch emulgierendes Wachs, Candelillawachs, Stearylalkohl, Cetylalkohol, Cetylstearylalkohol, Polyethylenglycol mit Mn von mehr als 3000 oder Kombinationen davon handelt; und
es sich bei dem Zerfallsbeschleuniger um vernetzte Natriumcarboxymethylcellulose, vernetztes Homopolymer aus N-Vinyl-2-pyrrolidon, modifizierte Stärke, Natriumcarboxymethylstärke, Natriumstärkeglycolat, Alginate, Stärke, Quellstärke oder Kombinationen davon handelt.

5. Komprimierte pharmazeutische Zusammensetzung nach Anspruch 1, wobei es sich bei dem wachsartigen Füllstoff um pulverisiertes Carnaubawachs und bei dem Zerfallsbeschleuniger um vernetzte Natriumcarboxymethylcellulose handelt.

6. Komprimierte pharmazeutische Zusammensetzung nach Anspruch 1, wobei die Menge des wachsartigen Füllstoffs 25 bis 75 Gew.-%, bezogen auf das Gesamtgewicht der komprimierten pharmazeutischen Zusammensetzung, beträgt.

7. Komprimierte pharmazeutische Zusammensetzung nach Anspruch 1, wobei die Menge des Zerfallsbeschleunigers 1 bis 5 Gew.-%, bezogen auf das Gesamtgewicht der komprimierten pharmazeutischen Zusammensetzung, beträgt.

8. Komprimierte pharmazeutische Zusammensetzung nach Anspruch 1, die außerdem einen zusätzlichen Hilfsstoff, einen zusätzlichen Zerfallsbeschleuniger, eine auf der Oberfläche der komprimierten Zusammensetzung aufgebrachte Beschichtung oder Kombinationen davon umfasst.

9. Komprimierte pharmazeutische Zusammensetzung nach Anspruch 8, wobei es sich bei dem zusätzlichen Hilfsstoff um ein Bindemittel, Schmiermittel, Fließregulierungsmittel, Verdichtungshilfsstoff, Farbstoff, Konservierungsmittel, Geschmacksstoff oder Kombinationen davon handelt.

10. Komprimierte pharmazeutische Zusammensetzung nach Anspruch 1, wobei das komprimierbare Gemisch außerdem einen Cellulosefüllstoff umfasst.

11. Komprimierte pharmazeutische Zusammensetzung nach Anspruch 1, wobei das komprimierbare Gemisch außerdem mikrokristalline Cellulose in einem Gewichtsverhältnis zu dem wachsartigen Füllstoff von 5:95 bis 40:60 (w/w) umfasst.

12. Komprimierte pharmazeutische Zusammensetzung nach Anspruch 1, wobei es sich bei dem pharmazeutischen Wirkstoff oder Salz davon um einen alpha-2-adrenergenen Wirkstoff, ein Analgetikum, einen Inhibitor des Angiotensin-konvertierenden Enzyms (ACE), ein Anxiolytikum, ein Antiarrhythmetikum, ein Antibakterium, ein Antibiotikum, ein Antidepressivum, ein Antidiabetikum, ein Antiemetikum, ein Antiepileptikum, ein Antimykotikum, ein Anthelminthikum, ein Antihistamin, ein Antihyperlipidemikum, ein Antihypertensivum, ein Antiinfektivum, ein Malariamittel, ein Antimikrobiotikum, ein Migränemittel, ein Antimuskarinikum, ein Antineoplastikum, ein Antiprotozoikum, ein Antipsychotikum, ein Antispasmodikum, ein Virostatikum, einen Wirkstoff gegen Aufmerksamkeitsdefizit-Hyperaktivitätsstörung (ADHS), einen β-Blocker, einen Calciumkanalblocker, ein Chemotherapeutikum, einen Cholinesterase-Inhibitor, einen COX-2-Inhibitor, ein Dekongestivum, ein Diuretikum, einen Histamin-2-Rezeptorantagonisten, ein Hypnotikum, ein blutdrucksenkendes Mittel, ein Immunsuppressivum, einen lipotropen Wirkstoff, ein Neuroleptikum, ein Opiodanalgetikum, einen peripheren Vasodilatator/Vasokonstriktor, ein Sedativum, einen Serotoninrezeptoragonisten oder pharmazeutisch akzeptable Kombinationen davon handelt.

13. Komprimierte pharmazeutische Zusammensetzung nach Anspruch 1, wobei es sich bei dem pharmazeutischen Wirkstoff um Amphetamin, Dextroamphetamin, Diltiazem, Fluvastatin, Hydromorphon, Morphin, Oxybutynin, Oxycodon, Paroxetin, Propranolol, Tolterodin, Venlafaxin oder pharmazeutisch akzeptable Salze, Solvate, Hydrate oder Polymorphe davon handelt.

14. Komprimierte pharmazeutische Zusammensetzung nach Anspruch 1, wobei die beschichteten Pellets eine Beschichtung umfassen, die hergestellt ist aus Alkylcellulose, Methylcellulose, Ethylcellulose, Hydroxypropylcellulose, Hydroxypropylmethylcellulose, Hydroxybutylmethylcellulose, Celluloseacetat, Cellulosepropionat, Celluloseacetatpropionat, Celluloseacetatbutyrat, Celluloseacetatphthalat, Carboxymethylcellulose, Cellulosetriacetat, Cellulosesulfat-Natriumsalz, Poly(methylmethacrylat), Poly(ethylmethacrylat), Poly(butylmethacrylat), Poly(isobutylmethacrylat), Poly(hexylmethacrylat), Poly(phenylmethacrylat), Poly(methylacrylat), Poly(isopropylacrylat), Poly(isobutylacrylat), Poly(octadecylacrylat), Poly(ethylen), Poly(ethylen) niedriger Dichte, Poly(ethylen) hoher Dichte, Poly(propylen), Poly(ethylenglycol), Poly(ethylenoxid), Poly(ethylenterephthalat), Poly(vinylalkohol), Polyvinyl(isobutylether), Poly(vinylacetat), Poly(vinylchlorid), Polyvinylpyrrolidon, oder Kombinationen davon; und wahlweise außerdem einen Weichmacher umfasst.

15. Komprimierte pharmazeutische Zusammensetzung nach Anspruch 1, wobei die beschichteten Pellets einen mittleren Durchmesser von 100 bis 3000 µm aufweisen.

16. Komprimierte pharmazeutische Zusammensetzung nach Anspruch 1, wobei das komprimierbare Gemisch Partikel mit einem mittleren Durchmesser von 0,1 bis 125 µm umfasst.

17. Komprimierte pharmazeutische Zusammensetzung nach Anspruch 1, wobei die komprimierte pharmazeutische Zusammensetzung einen Härtewert von wenigstens 8 Kilopond aufweist.

18. Komprimierte pharmazeutische Zusammensetzung nach Anspruch 1, wobei das Gewichtsverhältnis des wachsartigen Füllstoffs zum Zerfallsbeschleuniger 20:1 bis 45:1 beträgt.

19. Verfahren zur Herstellung einer komprimierten pharmazeutischen Zusammensetzung, umfassend:
Mischen eines pulverisierten, wachsartigen Füllstoffes und eines Zerfallsbeschleunigers zur Bildung eines komprimierbaren Gemisches mit einem mittleren Partikeldurchmesser von 0,1 bis 175 µm, wobei der wachsartige Füllstoff nicht-wasserlöslich ist und das Gewichtsverhältnis des wachsartigen Füllstoffs zum Zerfallsbeschleuniger 15:1 bis 50:1 beträgt,
mit der Maßgabe, dass das komprimierbare Gemisch nicht durch Extrusion, Spheronisation, Mischen unter hohen Scherkräften, Schmelzvermischung, Schmelzpelletierung, Gefriertrocknung oder einer Kombination davon hergestellt ist;
Mischen des komprimierbaren Gemisches mit einer Mehrzahl von beschichteten Pellets zur Bildung eines Pelletgemisches, wobei die beschichteten Pellets einen pharmazeutischen Wirkstoff oder ein Salz, Solvat, Hydrat oder Polymorph davon umfassen; und
Verdichten des Pelletgemisches mit einer konventionellen Direktkomprimieranlage, wobei die Druckkraft nicht mehr als etwa 25 kN beträgt.

20. Direktkomprimierbares Gemisch zur Bildung komprimierter pharmazeutischer Zusammensetzungen, umfassend:
ein Gemisch eines pulverisierten, wachsartigen Füllstoffs und eines Zerfallsbeschleunigers, wobei das Gewichtsverhältnis des wachsartigen Füllstoffs zum Zerfallsbeschleuniger 15:1 bis 50:1 beträgt, und gegebenenfalls außerdem einen Cellulosefüllstoff, ein Bindemittel, ein Schmiermittel, ein Fließregulierungsmittel, einen Verdichtungshilfsstoff, einen Farbstoff, ein Konservierungsmittel, einen Geschmackstoff oder Kombinationen davon umfassend,
wobei das Gemisch einen mittleren Partikeldurchmesser von 0,1 bis 175 µm aufweist, mit der Maßgabe, dass das komprimierbare Gemisch nicht durch Extrusion, Spheronisation, Mischen unter hohen Scherkräften, Schmelzvermischung, Schmelzpelletierung, Gefriertrocknung oder Kombinationen davon hergestellt ist,
wobei das komprimierbare Gemisch zur Direktkomprimierung von Gemischen des komprimierbaren Gemisches und beschichteten Arzneimittelpellets geeignet ist, im Wesentlichen ohne die beschichteten Arzneimittelpellets zu beschädigen.

21. Komprimierbares Gemisch nach Anspruch 20, wobei es sich bei dem pulverisierten wachsartigen Füllstoff um Carnaubawachs, pflanzliches Wachs, Fruchtwachs, mikrokristallines Wachs, Bienenwachs, Kohlenwasserstoffwachs, Parrafinwachs, Cetylesterwachs, nicht-ionisch emulgierendes Wachs, anionisch emulgierendes Wachs, Candelillawachs, Stearylalkohl, Cetylalkohol, Cetylstearylalkohol, Polyethylenglycol mit Mn von mehr als 3000 oder Kombinationen davon handelt; und
es sich bei dem Zerfallsbeschleuniger um vernetzte Natriumcarboxymethylcellulose, vernetztes Homopolymer aus N-Vinyl-2-pyrrolidon, modifizierte Stärke, Natriumcarboxymethylstärke, Natriumstärkeglycolat, Alginate, Stärke, Quellstärke oder Kombinationen davon handelt.

22. Komprimierbares Gemisch nach Anspruch 20, wobei das komprimierbare Gemisch zur Direktkomprimierung von Gemischen des komprimierbaren Gemisches und beschichteten Arzneimittelpellets zur Bildung einer komprimierten pharmazeutischen Zusammensetzung geeignet ist, wobei das Freisetzungsprofil der komprimierten pharmazeutischen Zusammensetzung im Wesentlichen das Gleiche ist wie das Freisetzungsprofil der beschichteten Arzneimittelpellets allein.

23. Komprimierbares Gemisch nach Anspruch 22, wobei es sich bei dem pulverisierten wachsartigen Füllstoff um einen nicht-wasserlöslichen pulverisierten wachsartigen Füllstoff handelt.

24. Komprimierbares Gemisch nach Anspruch 22, wobei das Gewichtsverhältnis des wachsartigen Füllstoffs zum Zerfallsbeschleuniger 20:1 bis 45:1 beträgt.

25. Komprimierbares Gemisch nach Anspruch 22, wobei das Gewichtsverhältnis des wachsartigen Füllstoffs zum Zerfallsbeschleuniger 25:1 bis 40:1 beträgt.

26. Komprimierbares Gemisch nach Anspruch 22, wobei das Gemisch des pulverisierten wachsartigen Füllstoffs und des Zerfallsbeschleunigers als Pulver vorliegt.

27. Komprimierbares Gemisch nach Anspruch 22, wobei das komprimierbare Gemisch außerdem einen Cellulosefüllstoff umfasst.

28. Komprimierbares Gemisch nach Anspruch 22, wobei das komprimierbare Gemisch außerdem mikrokristalline Cellulose oder pulverisierte Cellulose in einem Gewichtsverhältnis zum wachsartigen Füllstoff von 5:95 bis 40:60 (w/w) umfasst.

## Revendications

1. Composition pharmaceutique comprimée, qui comprend :
une pluralité de granules enrobés, les granules enrobés comprenant un agent pharmaceutiquement actif ou un sel, un solvate, un hydrate, ou un polymorphe de celui-ci ; et
un mélange compressible, le mélange compressible comprenant i) une charge cireuse non hydrosoluble et ii) un désintégrant, le rapport en poids de la charge cireuse au désintégrant étant de 15/1 à 50/1 et le mélange compressible se trouvant sous une forme pulvérulente avec un diamètre moyen de particules de 0,1 à 175 micromètres,
à condition que le mélange compressible ne soit pas préparé par extrusion, sphéronisation, mélange sous cisaillement élevé, mélange en fusion, granulation par fusion, lyophilisation, ou une combinaison de ceux-ci ;
la composition comprimée présentant un profil de dissolution in vitro selon une méthode de la pharmacopée US qui est sensiblement la même que la dissolution des granules enrobés en l'absence du mélange compressible.

2. Composition pharmaceutique comprimée selon la revendication 1, la biodisponibilité de l'agent pharmaceutiquement actif de la composition comprimée étant sensiblement la même, après l'administration orale de la composition à un mammifère, que la biodisponibilité de l'agent pharmaceutiquement actif obtenue par l'administration des granules enrobés en l'absence du mélange compressible.

3. Composition pharmaceutique comprimée selon la revendication 1, la méthode de la pharmacopée US étant USP 28, <711> Dissolution, appareil 2, pale, vitesse des pales de 100 tr/minute et 900 mL de HCl 0,1N en tant que milieu de dissolution à 37°C.

4. Composition pharmaceutique comprimée selon la revendication 1, la charge cireuse étant de la cire de carnauba, de la cire végétale, de la cire de fruits, de la cire microcristalline, de la cire d'abeille, de la cire d'hydrocarbures, de la cire de paraffine, de la cire à base d'esters cétyliques, de la cire émulsifiante non ionique, de la cire émulsifiante anionique, de la cire de candelilla, de l'alcool stéarylique, de l'alcool cétylique, de l'alcool cétoastéarylique, du polyéthylène glycol ayant une Mn supérieure à 3000, ou des combinaisons de ceux-ci ; et
le désintégrant étant la carboxyméthylcellulose sodique réticulée, un homopolymère réticulé de N-vinyl-2-pyrrolidone, des amidons modifiés, le carboxyméthyl amidon sodique, le glycolate d'amidon sodique, les alginates, l'amidon, l'amidon prégélatinisé, ou des combinaisons de ceux-ci.

5. Composition pharmaceutique comprimée selon la revendication 1, la charge cireuse étant la cire de carnauba pulvérulente et le désintégrant étant la carboxyméthylcellulose sodique réticulée.

6. Composition pharmaceutique comprimée selon la revendication 1, la quantité de charge cireuse étant de 25 à 75 % en poids en se basant sur le poids total de la composition pharmaceutique comprimée.

7. Composition pharmaceutique comprimée selon la revendication 1, la quantité de désintégrant étant de 1 à 5 % en poids en se basant sur le poids total de la composition pharmaceutique comprimée.

8. Composition pharmaceutique comprimée selon la revendication 1, comprenant en outre un excipient supplémentaire, un désintégrant supplémentaire, un enrobage disposé sur la surface de la composition comprimée, ou des combinaisons de ceux-ci.

9. Composition pharmaceutique comprimée selon la revendication 8, l'excipient supplémentaire étant un liant, un lubrifiant, un agent de glissement, un adjuvant de compression, un colorant, un conservateur, un agent aromatisant, ou des combinaisons de ceux-ci.

10. Composition pharmaceutique comprimée selon la revendication 1, le mélange compressible comprenant en outre une charge cellulosique.

11. Composition pharmaceutique comprimée selon la revendication 1, le mélange compressible comprenant en outre de la cellulose microcristalline en un rapport en poids à la charge cireuse de 5/95 à 40/60 (p/p).

12. Composition pharmaceutique comprimée selon la revendication 1, l'agent pharmaceutiquement actif ou un sel de celui-ci étant un agent alpha-2-adrénergique, un analgésique, un inhibiteur de l'enzyme de conversion de l'angiotensine (ACE), un agent anti-anxiété, un antiarythmique, un antibactérien, un antibiotique, un antidépresseur, un antidiabétique, un antiémétique, un antiépileptique, un antifongique, un antihelminthique, un antihistaminique, un anti-hyperlipidémiant, un agent hypotenseur, un antiinfectieux, un antipaludéen, un antimicrobien, un antimigraineux, un agent antimuscarinique, un agent antinéoplasique, un agent anti-protozoaire, un agent antipsychotique, un antispasmodique, un agent antiviral, un agent contre les troubles déficitaires de l'attention avec hyperactivité (ADHD), un β-bloquant, un bloquant des canaux calciques, un agent chimiothérapeutique, un inhibiteur de la cholinestérase, un inhibiteur de Cox-2, un décongestionnant, un diurétique, un antagoniste du récepteur 2 de l'histamine, un hypnotique, un agent hypotenseur, un immunosuppresseur, un agent lipotropique, un agent neuroleptique, un analgésique opioïde, un vasodilatateur/vasoconstricteur périphérique, un sédatif, un agoniste des récepteurs de la sérotonine, ou des combinaisons pharmaceutiquement acceptables de ceux-ci.

13. Composition pharmaceutique comprimée selon la revendication 1, l'agent pharmaceutiquement actif étant une amphétamine, une dextroamphétamine, le diltiazem, la fluvastatine, l'hydromorphone, la morphine, l'oxybutynine, l'oxycodone, la paroxetine, le propranolol, la toltérodine la venlafaxine, ou un sel, un solvate, un hydrate ou un polymorphe pharmaceutiquement acceptable de ceux-ci.

14. Composition pharmaceutique comprimée selon la revendication 1, les granules enrobés comprenant un enrobage préparé à partir d'alkyl cellulose, de méthylcellulose, d'éthylcellulose, d'hydroxypropyl cellulose, d'hydroxypropyl méthyl cellulose, d'hydroxybutyl méthyl cellulose, d'acétate de cellulose, de propionate de cellulose, d'acétate propionate de cellulose, d'acétate butyrate de cellulose, d'acétate phtalate de cellulose, de carboxyméthyl cellulose, de triacétate de cellulose, de sel de sulfate de cellulose sodique, de poly(méthacrylate de méthyle), de poly(méthacrylate d'éthyle), de poly (méthacrylate de butyle), de poly(méthacrylate d'isobutyle), de poly(méthacrylate d'hexyle), de poly(méthacrylate de phényle), de poly (acrylate de méthyle), de poly (acrylate d'isopropyle), de poly (acrylate d'isobutyle), de poly (acrylate d'octadécyle), de (poly)éthylène, de poly(éthylène) de faible densité, de poly(éthylène) de haute densité, de (poly propylène), de poly(éthylène glycol), de poly(oxyde d'éthylène), de poly(téréphtalate d'éthylène), d'un poly(alcool vinylique), de poly(éther vinyl isobutylique), de poly(acétate de vinyle), de poly(chlorure de vinyle), de polyvinylpyrrolidone, ou de combinaisons de ceux-ci et comprenant en outre, facultativement, un plastifiant.

15. Composition pharmaceutique comprimée selon la revendication 1, les granules enrobés ayant un diamètre moyen de 100 à 3 000 micromètres.

16. Composition pharmaceutique comprimée selon la revendication 1, le mélange compressible comprenant des particules ayant un diamètre moyen de 0,1 à 125 micromètres.

17. Composition pharmaceutique comprimée selon la revendication 1, la composition pharmaceutique comprimée ayant une valeur de dureté d'au moins 8 kilogrammes-poids.

18. Composition pharmaceutique comprimée selon la revendication 1, le rapport en poids de la charge cireuse au désintégrant étant de 20/1 à 45/1.

19. Procédé de fabrication d'une composition pharmaceutique comprimée, qui comprend :
le mélange d'une charge cireuse pulvérulente et d'un désintégrant pour former un mélange compressible avec un diamètre de particules moyen de 0,1 à 175 micromètres, la charge cireuse n'étant pas hydrosoluble et le rapport en poids de la charge cireuse au désintégrant étant de 15/1 à 50/1,
à condition que le mélange compressible ne soit pas préparé par extrusion, sphéronisation, mélange sous cisaillement élevé, mélange en fusion, granulation par fusion, lyophilisation, ou une combinaison de ceux-ci ;
le mélange du mélange compressible avec une pluralité de granules enrobés pour former un mélange de granules, les granules enrobés comprenant un agent pharmaceutiquement actif ou un sel, un solvate, un hydrate, ou un polymorphe de celui-ci ; et
la compression du mélange de granules avec des équipements de compression directe, la force de compression n'étant pas supérieure à environ 25 kilonewtons.

20. Mélange compressible par compression directe pour former des compositions pharmaceutiques comprimées, qui comprend :
un mélange d'une charge cireuse pulvérulente et d'un désintégrant, le rapport en poids de la charge cireuse au désintégrant étant de 15/1 à 50/1, et comprenant en outre, facultativement, une charge cellulosique, un liant, un lubrifiant, un agent de glissement, un adjuvant de compression, un colorant, un conservateur, un agent aromatisant, ou des combinaisons de ceux-ci ;
le mélange ayant un diamètre moyen de particules de 0,1 à 175 micromètres, à condition que le mélange compressible ne soit pas préparé par extrusion, sphéronisation, mélange sous cisaillement élevé, mélange en fusion, granulation par fusion, lyophilisation, ou une combinaison de ceux-ci ;
le mélange compressible étant adapté pour comprimer directement des mélanges du mélange compressible et des granules médicamenteux enrobés sensiblement sans endommager les granules médicamenteux enrobés.

21. Mélange compressible selon la revendication 20, la charge cireuse pulvérulente étant de la cire de carnauba, de la cire végétale, de la cire d'abeille, de la cire microcristalline, de la cire d'abeille, de la cire d'hydrocarbures, de la cire de paraffine, de la cire à base d'esters cétyliques, de la cire émulsifiante non ionique, de la cire émulsifiante anionique, de la cire de candelilla, de l'alcool stéarylique, de l'alcool cétylique, de l'alcool cétoastéarylique, du polyéthylène glycol ayant une Mn supérieure à 3000, ou des combinaisons de ceux-ci ; et
le désintégrant étant la carboxyméthylcellulose sodique réticulée, un homopolymère réticulé de N-vinyl-2-pyrrolidone, des amidons modifiés, le carboxyméthyl amidon sodique, le glycolate d'amidon sodique, les alginates, l'amidon, l'amidon prégélatinisé, et des combinaisons de ceux-ci.

22. Mélange compressible selon la revendication 20, le mélange compressible étant adapté à la compression directe de mélanges du mélange compressible et de granules médicamenteux enrobés pour former une composition pharmaceutique comprimée, le profil de dissolution présenté par la composition pharmaceutique comprimée étant sensiblement le même que le profil de dissolution des granules médicamenteux seuls.

23. Mélange compressible selon la revendication 22, la charge cireuse pulvérulente étant une charge cireuse pulvérulente non hydrosoluble.

24. Mélange compressible selon la revendication 22, le rapport en poids de la charge cireuse au désintégrant étant de 20/1 à 45/1.

25. Mélange compressible selon la revendication 22, le rapport en poids de la charge cireuse au désintégrant étant de 25/1 à 40/1.

26. Mélange compressible selon la revendication 22, le mélange de la charge cireuse pulvérulente et du désintégrant étant sous la forme d'une poudre.

27. Mélange compressible selon la revendication 22, le mélange compressible comprenant en outre une charge de cellulose.

28. Mélange compressible selon la revendication 22, le mélange compressible comprenant en outre de la cellulose microcristalline ou de la cellulose en poudre en un rapport en poids à la charge cireuse de 5/95 à 40/60 (p/p).
